(19) Europäisches Patentamt
European Patent Office
Office européen des brevets

(11) EP 0 466 569 B1

(12) # FASCICULE DE BREVET EUROPEEN

(45) Date de publication et mention de la délivrance du brevet: **17.04.1996 Bulletin 1996/16**

(21) Numéro de dépôt: **91401891.6**

(22) Date de dépôt: **08.07.1991**

(51) Int Cl.[6]: **C07D 495/04**, C07D 333/20, A61K 31/435 // (C07D495/04, 333:00, 221:00)

(54) **Procédé de préparation d'un dérivé N-phénylacétique de tétrahydro-thiéno (3,2-c) pyridine et son intermédiaire de syntèse**

Verfahren zur Herstellung eines N-Phenylacetylderivats von Tetrahydrothieno[3,2-c]pyridin und Zwischenprodukt der Synthese

Process for preparation of N-phenylacetyl derivative of tetrahydrothieno[3,2-c]pyridine and intermediate of synthesis

(84) Etats contractants désignés:
**AT BE CH DE DK ES FR GB GR IT LI LU NL SE**

(30) Priorité: **10.07.1990 FR 9008749**

(43) Date de publication de la demande: **15.01.1992 Bulletin 1992/03**

(73) Titulaire: **SANOFI**
**F-75008 Paris (FR)**

(72) Inventeurs:
- **Descamps, Marcel**
  **F-31600 Lhum (FR)**
- **Radisson, Joel**
  **F-31100 Toulouse (FR)**

(74) Mandataire: **Polus, Camille et al**
**c/o Cabinet Lavoix**
**2, Place d'Estienne d'Orves**
**F-75441 Paris Cedex 09 (FR)**

(56) Documents cités:

| | |
|---|---|
| **EP-A- 0 000 453** | **EP-A- 0 099 802** |
| **EP-A- 0 281 459** | **EP-A- 0 321 349** |
| **EP-A- 0 342 118** | **US-A- 4 127 580** |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1)Convention sur le brevet européen).

EP 0 466 569 B1

## Description

La présente invention concerne un procédé de synthèse stéréospécifique pour la préparation de l'alpha-(tétrahydro-4,5,6,7 thiéno[3,2--c]pyridyl-5)(chloro-2 phényl)acétate de méthyle, dont l'isomère dextrogyre, le clopidogrel, est connu pour son intérêt en thérapeutique notamment pour ses activités antiagrégante plaquettaire et antithrombotique.

Un procédé de préparation du mélange racémique, par substitution de la tétrahydro-4,5,6,7 thiéno[3,2-c]pyridine, est décrit dans EP-A-99802, tandis que l'isolement de chacun des stéréoisomères par recristallisation des sels avec respectivement les acides camphosulfoniques dextrogyre et lévogyre est décrit dans EP-A-O 281 459.

EP 0 000 453 divulgue un procédé de préparation de dérivés tétrahydro-thiéno-pyridines éventuellement N-substituées par un groupe arylalkyle.

Selon ce procédé une 2-(2-thiényl)-éthylamine N-substituée réagit avec un composé de formule X-CH($R_4$)-Y. On notera cependant que EP-0 000 453 ne décrit nullement un procédé de synthèse stéréospécifique pour la préparation de tétrahydro-thiéno-pyridines optiquement actives.

EP-4 127 580 vise de même un procédé de préparation de tétrahydro-thiéno-pyridines comprenant la réaction du formaldéhyde sur des 2-(2-thiényl)éthylamine N-substituées.

Toutefois, ce document ne concerne pas davantage la synthèse stéréospécifique de tels composés.

On a maintenant trouvé un procédé de préparation qui permet d'obtenir, avec d'excellents rendements, uniquement l'un des énantiomères. En effet, le procédé est stéréospecifique, et il n'y a pas de racémisation du carbone asymétrique, alors que l'on a constaté qu'en appliquant le procédé décrit dans EP-A-99802 précédemment mentionné, c'est-à-dire en faisant réagir l'un des énantiomères de l'alpha(chloro-2 phényl)acétate de méthyle sur la tétrahydro-4,5,6,7 thiéno [3,2-c]pyridine, on obtient un mélange du clopidogrel et de son énantiomère lévogyre de formule

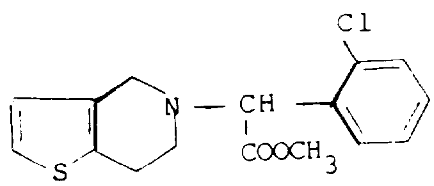

I

Le procédé selon l'invention consiste à faire réagir un agent de formylation sur l'isomère dextrogyre du composé de formule

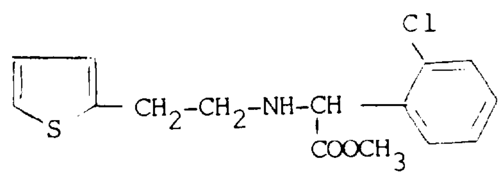

II

et à cycliser les composés intermédiaires formés en présence d'un acide.

Les agents de formylation sont a) l'aldéhyde formique et les composés généralement connus pour le libérer sous forme réactive, tels que ses dérivés de polymérisation, son hydrate, b) les composés de formule $XCH_2Y$ dans laquelle X représente un atome d'halogène, un groupe alkoxy en $C_1$ à $C_4$, un groupe alkylthio en $C_1$ à $C_4$ ou un groupe amino et Y représente un groupe alkoxy en $C_1$ à $C_4$, alkylthio en $C_1$ à $C_4$, amino, alkoxycarbonyle en $C_2$ à $C_5$ ou phénoxycarbonyle; c) et les composés hétérocycliques de formule

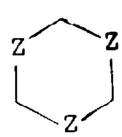

dans laquelle Z représente O, NH ou S, tel que le s-trioxane.

On peut effectuer les deux étapes de la réaction successivement, éventuellement en isolant les composés intermédiaires, ou simultanément.

On considère qu'une hydroxyméthylèneamine ou un hétérocycle de type triméthylènetriamine peuvent notamment se former comme composés intermédiaires, mais l'invention ne saurait être limitée par ces hypothèses.

Lorsque les réactions sont successives, la première étape peut être effectuée dans l'eau ou dans un alcool, éventuellement en présence d'un solvant hydrocarboné, tel que le benzène, le toluène ou l'éther de pétrole, d'un solvant halogéné tel que le chlorure de méthylène, ou d'un éther; la cyclisation est ensuite effectuée dans un solvant polaire,

tel que l'eau, un alcool ou le diméthylformamide, ou dans un mélange de ces solvants.

On préfère comme agent de formylation, l'aldéhyde formique, qui pourra être introduit dans le milieu en solution aqueuse. L'acide peut être un acide organique ou minéral, en général fort tel que l'acide sulfurique ou un acide halogénhydrique comme l'acide chlorhydrique, ou un acide sulfonique comme l'acide méthanesulfonique.

Lorsque les réactions sont effectuées simultanément, le milieu réactionnel est un solvant polaire, tel que l'eau ou un alcool, et l'acide minéral ou organique est introduit dans le milieu, de préférence en quantité stoechiométrique par rapport au composé de formule II mis en oeuvre; l'acide est dans ce dernier cas un acide fort qui peut être simplement introduit dans le milieu sous forme de son sel avec le composé de formule II. On peut aussi utiliser un solvant acide, tel que l'acide formique ou l'acide acétique, le premier associé au paraformaldéhyde étant particulièrement préféré.

L'isomère dextrogyre du composé de formule II et ses sels, nécessaires à la mise en oeuvre du procédé, sont un autre objet de l'invention .

Ils peuvent être préparés par action de l'alpha-amino(chloro-2phényl) acétate de méthyle de formule IV sur un dérivé thiénylique de formule III selon le schéma réactionnel (a) :

(a)

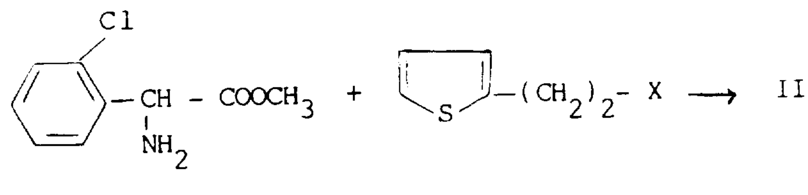

IV III

dans lequel X représente un atome d'halogène, notamment Br, ou un groupe sulfonique $RSO_2O$- dans lequel R représente un groupe alkyle en $C_1$ à $C_4$ ou un groupe phényle, éventuellement substitué.

On préfère utiliser le dérivé p-toluènesulfonique, plus stable.

Les conditions de la réaction sont classiques; on maintient à une température comprise entre 50°C et 100°C, pendant 10 à 30 heures, les composés dans un solvant inerte, de préférence en présence d'une base qui neutralise l'acide formé. Comme base, on peut utiliser une base organique, comme une amine tertiaire et plus particulièrement la triéthylamine, ou une base minérale comme les carbonates alcalins, tel que $Na_2CO_3$ ou $NaHCO_3$, ou les phosphates alcalins, tels que $K_2HPO_4$. On peut utiliser comme milieu réactionnel de nombreux solvants organiques, dont les alcools, comme le méthanol, les cétones, comme la méthyléthylcétone, les éthers, comme le tétrahydrofuranne, les nitriles, comme l'acétonitrile, les esters, comme l'acétate d'éthyle ou les hydrocarbures, halogénés ou non, comme le toluène ou le chlorure de méthylène; on a toutefois constaté que si l'on veut obtenir un seul des énantiomères du composé II en faisant réagir l'un des énantiomères du composé IV au lieu du mélange racémique, le choix du solvant n'était pas indifférent, une racémisation partielle ayant lieu dans certains solvants; les esters permettent d'éviter la racémisation et on préfère utiliser l'acétate de méthyle comme solvant lorsque l'on fait réagir l'isomère dextrogyre du composé IV (chlorhydrate :$[\alpha]_D^{20}$ = + 115°C = 1,$CH_3OH$) pour obtenir l'isomère dextrogyre du composé II ($[\alpha]_D^{20}$ = + 98°C; C = O,76,$CH_3OH$) qui, en appliquant le procédé de l'invention, donne le clopidogrel (chlorhydrate dextrogyre : $[\alpha]_D^{20}$ = + 65°; C = 1,$CH_3OH$).

On peut aussi préparer le composé de formule II à partir de la thiényléthylamine, selon le schéma réactionnel (b) :

(b)

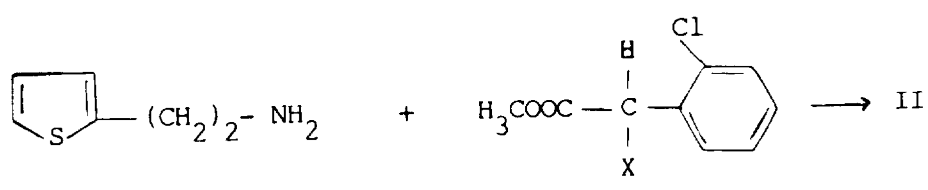

V

dans lequel X représente Cl ou Br.

La réaction est effectuée dans un solvant inerte, éventuellement en présence d'une base pour salifier l'acide formé. Par exemple, lorsque X = Br on opère avantageusement dans le méthanol en présence d'un carbonate alcalin.

Les produits mis en réaction selon (a) et (b) sont connus ou peuvent être préparés par analogie à des procédés connus.

Par exemple, la préparation de la thiényléthylamine est décrite dans FR-A-2 608 607, celle du composé III dans

FR-A-2 300 090, celle du composé V racémique (X = Br) dans Chem. Pharm. Bull. 30 (10) 3601-3616 (1982); le composé IV peut être préparé par exemple par estérification de l'aminoacide racémique ou de chacun des énantiomères par réaction avec du chlorure de thionyle et du méthanol à température inférieure à 5°C, selon un procédé classique; l'acide précurseur de IV est décrit dans J. Chem. Soc. p. 1440-1444 (1962) : ces deux isomères peuvent être séparés de manière classique par recristallisation ou par une méthode mettant en jeu une réaction enzymatique. Les deux énantiomères de IV peuvent aussi être séparés par recristallisation avec les énantiomères de l'acide tartrique en présence ou non de méthyléthylcétone.

Les énantiomères du composé II sont aussi obtenus par recristallisation du sel du composé racémique avec un acide optiquement actif tel que les acides (+) ou (-) tartrique dans l'isopropanol ou les acides (+) ou (-) campho-10 sulfonique dans l'acétone.

Dans ce qui suit, on décrit des exemples de réalisation du procédé selon l'invention et des exemples de préparation des intermédiaires de synthèse racémique et de leurs énantiomères. Les points de fusion mentionnés ont été déterminés en tube capillaire. Les concentrations des solutions pour la mesure des pouvoirs rotatoires sont exprimées en g/100 ml.

Préparation des alpha-amino (chloro-2phényl) acétates de méthyle (IV)

a) racémique :

A température inférieure à -10°C, on introduit 330 g de chlorure de thionyle dans 750 ml de méthanol, puis rapidement 245 g d'acide alpha-amino (chloro-2 phényl)acétique; après 48 heures d'agitation à température ambiante, les produits volatils sont éliminés sous pression réduite; le résidu, dissous dans 1000 ml de méthanol est filtré sur charbon actif et le chlorhydrate du produit cherché est précipité dans un excès d'éther isopropylique.

Après séchage, on obtient 290g du chlorhydrate de l'amino ester IV racémique qui fond à 198°C.

L'amino ester correspondant, libéré de son sel par action de $NaHCO_3$ dans l'eau et le dichloro-1,2 éthane distille entre 96°C et 100°C sous 40 Pa.

b) énantiomère dextrogyre :

α-1- On introduit dans une solution de 64 g d'acide alpha-amino(chloro-2phényl)acétique dans 1,1 l d'eau au reflux, 80 g d'acide (+) campho-10 sulfonique et on laisse cristalliser plusieurs jours à température ambiante. Le précipité apparu est isolé et' le filtrat concentré jusqu'à 150 ml. Le précipité formé est réuni au premier et l'ensemble est recristallisé dans l'eau.

On obtient ainsi 45g de (+) camphosulfonate de (+) acide alpha-amino(chloro-2phényl)acétique

$[\alpha]_D^{22}$ = + 92° (C = 1, HCl N).

α-2- estérification :

On libère la base du sel obtenu selon 1 par action de $NaHCO_3$ et on en introduit 24,5 g entre -20°C et -8°C dans 75 ml de méthanol auxquels on a ajouté 33 g de chlorure de thionyle. On laisse revenir le mélange à température ambiante, et le maintient pendant 48 heures, sous agitation, avant de distiller les produits volatils sous pression réduite. Le résidu est dissous dans 100 ml de méthanol et on verse cette solution dans 800 ml d'éther isopropylique pour précipiter le chlorhydrate du (+) alpha-amino(chloro-2 phényl)acétate de méthyle.

β - On introduit dans une solution de 62 g de l'aminoester obtenu selon a) dans 270 ml de méthanol au reflux, 45 g d'acide (+) tartrique puis 1,2 l d'acétonitrile et 25 ml de méthyléthylcétone vers 60°C. Le mélange est maintenu une journée vers 60°C et le solide est filtré à 50°C puis redissous dans un mélange au reflux de 400 ml d'acétonitrile et de 100 ml de méthanol; le précipité formé est séparé à 45°c. Après séchage, on obtient 49 g de (+) tartrate de (+)alpha-amino (chloro-2phényl)acétate de méthyle $[\alpha]_D^{20}$= + 85,5° (C=1,$CH_3OH$).

Préparation de l'alpha-chloro-(chloro-2phényl) acétate de méthyle racémique (V) :

On mélange 93,3 g d'acide chloro-2 mandélique avec 208 g de $PCl_5$ et on porte lentement jusqu'à 60°C, température à laquelle la réaction exothermique démarre; le mélange est alors maintenu environ 2 heures entre 120°C et 130°C jusqu'à la fin de dégagement de HCl; les produits volatils sont alors éliminés sous pression réduite et le résidu est dissous dans 200 ml de méthanol. Le mélange est maintenu 2 heures à sa température de reflux, le méthanol est évaporé puis le résidu est dissous dans $CH_2Cl_2$. La phase organique est lavée à l'eau puis le solvant est évaporé; le résidu huileux est distillé sous pression réduite E = 85°C/2000 Pa.

On obtient ainsi 50 g de l'ester alpha chloré recherché.

EXEMPLE 1

Alpha-(thiényl-2éthylamino)(chloro-2phényl)acétate de méthyle racémique (II -schéma a).

On libère de son chlorhydrate 9,6 g d'alpha-amino(chloro-2 phényl)acétate de méthyle par action de 7 g de $NaHCO_3$ en présence de 150 ml de $CH_2Cl_2$ et 50 ml d'eau.

L'amine obtenue est mise en solution dans 50 ml d'acétonitrile; on introduit alors 4,8 g de $NaHCO_3$ et 12 g de para-toluènesulfonate de thiényl-2 éthyle (III $X=CH_3C_6H_4SO_3$); le mélange est maintenu pendant 22 heures à 80°C, puis les produits volatils évaporés sous pression réduite. Le résidu est dissous dans 150 ml d'acétate d'éthyle et 50 ml d'eau; la phase organique est décantée et on y introduit vers 5°C, 6 g d'une solution aqueuse concentrée d'acide chlorhydrique, en mélange avec 15 g de glace pilée. Le précipité formé sous agitation est filtré et séché. On obtient ainsi 9,4 g du chlorhydrate du composé de formule II. F = 175°C.

EXEMPLE 2

Alpha-(thiényl-2éthylamino)(chloro-2phényl)acétate de méthyle dextrogyre (II -schéma a).

3,46 g (+)alpha-amino(chloro-2phényl)acétate de méthyle, obtenus par action de $NaHCO_3$ dans un mélange d'eau et de chlorure de méthylène sur le (+) tartrate de (+) alpha-amino(chloro-2phényl)acétate de méthyle, sont introduits dans 50 ml d'acétate de méthyle, ainsi que 4 g de $K_2HPO_4$ et 6 g de para-toluènesulfonate de (thiényl-2)éthyle et le mélange est maintenu vers 80°C pendant 40 heures. Les produits volatils sont alors éliminés sous pression réduite et. le résidu dissous dans un mélange de 75 ml d'acétate d'éthyle et 40 ml d'eau. La phase organique est décantée, et concentrée de moitié. On y introduit ensuite, vers 5°c, 10 g de glace pilée avec 3 ml d'une solution aqueuse d'acide chlorhydrique concentrée.

Le précipité formé est isolé et séché.

On obtient ainsi 3 g du chlorhydrate du produit cherché.

$[\alpha]_D^{20}$ = + 104° (C = 1,$CH_3OH$).

On obtient le même produit avec un rendement légèrement inférieur en remplaçant les 6 g de para-toluènesulfonate de (thiényl-2)éthyle par 4,2 g de (bromo-2)éthyl-2 thiophène.

EXEMPLE 3

Alpha-(thiényl-2éthylamino)(chloro-2phényl)acétate de méthyle dextrogyre par dédoublement du racémique.

a) 175 g du chlorhydrate du composé II obtenus selon l'exemple 1 sont dissous dans un mélange de 0,75 l de chlorure de méthylène et de 0,25 l d'eau. On ajoute alors progressivement 45g de $NaHCO_3$ et sépare la phase organique après agitation et décantation. après les traitements habituels, on obtient l'aminoester que l'on dissout dans 850 ml d'acétone et on introduit dans cette solution 87 g d'acide (+) campho-10 sulfonique; après 12 heures à température ambiante, le précipité formé est isolé.

On obtient ainsi 146,5 g de camphosulfonate.

$[\alpha]_D^{22}$ = + 51,7° (C = 1,$CH_3OH$).

Le camphosulfonate est mis en suspension dans 700 ml d'acétone au reflux et on ajoute 300 ml de méthyléthylcétone pour obtenir la solubilisation complète. Le précipité formé après retour à température ambiante est isolé puis retraité à chaud par 500 ml d'acétone et 300 ml de méthyléthylcétone pour donner 95 g de (+)camphosulfonate du produit cherché.

F = 95°C. $[\alpha]_D^{22}$ = +82° (C = 1,$CH_3OH$).

b) 33,5 g du composé II sont introduits dans 500 ml d'isopropanol avec 14,6 g d'acide (+) tartrique; on porte à 50°C puis on abandonne le mélange à température ambiante. Le précipité formé est isolé et recristallisé 4 fois dans l'isopropanol. On obtient finalement le (+) tartrate du produit cherché dextrogyre qui fond à 105°C.

Le pouvoir rotatoire de l'amine est $[\alpha]_D^{20}$ = + 99,76° (C = 1,$CH_3OH$).

EXEMPLE 4

Alpha-(thiényl-2éthylamino)(chloro-2phényl)acétate de méthyle racémique (II -schéma b).

On introduit dans 300 ml de méthanol, 543 g d'acide alpha-bromo (chloro-2phényl)acétique et 30 g d'acide sulfurique concentré. On porte le mélange 3 heures à sa température de reflux et après avoir refroidi vers 30°C, on ajoute progressivement 230 g de $KHCO_3$ et 265 g de (thiényl-2)-2 éthylamine. Le mélange est maintenu 1 heure 30 à sa température de reflux, puis le solvant est évaporé sous vide. On verse sur le résidu 1 l d'eau et 1 l d'acétate d'éthyle et on sépare la phase organique après agitation et décantation. On y introduit alors 300 ml de glace et 180 ml d'acide chlorhydrique concentré. Le produit précipité est filtré et séché. On obtient ainsi 641 g du chlorhydrate du produit cherché qui fond à 175°C.

EXEMPLE 5

Alpha-(tétrahydro-4,5,6,7thiéno[3,2-c]pyridyl-5)(chloro-2phényl)acétate de méthyle dextrogyre (I).

a) 38 g de (+)camphosulfonate de (+)alpha-(thiényl-2éthylamino)(chloro-2phényl)acétate de méthyle sont traités par 7g de $NaHCO_3$ dans un mélange de200 ml de dichloro-1,2 éthane et 70 ml d'eau. Après agitation et décantation, la phase organique séchée est concentrée.

Le résidu est dissous dans 90 ml d'acide formique anhydre avec 3,8 g de paraformaldéhyde et le mélange est chauffé 20 min à 50°C. On élimine les produits volatils sous pression réduite et verse sur le résidu 250 ml de dichloro-1,2 éthane et 400 ml d'eau. Après agitation et décantation, le solvant de la phase organique isolée est évaporé et l'huile résiduelle est dissoute dans 250 ml d'éther éthylique. Le chlorhydrate du produit final précipite par addition d'éther éthylique saturé de HCl, dans la solution.

Le précipité isolé et séché, pèse 21 g; il fond vers 130°-140°C. $[\alpha]_D^{26}$ = + 63° (C = 1,$CH_3OH$).

b) On maintient pendant 1 heure 15 minutes à 80°C, une solution de 16 g de (+)camphosulfonate de (+)alpha-(thiényl-2éthylamino)(chloro-2phényl)acétate de méthyle dans 50 ml d'une solution aqueuse à 37% (p/p) d'aldéhyde formique. On introduit ensuite dans le milieu, à température ambiante, 100 ml d'eau et 200 ml de chlorure de méthylène et du $NaHCO_3$ jusqu'à un pH voisin de 8. La phase organique séparée est lavée à l'eau, séchée et concentrée. On obtient ainsi 9,75 g d'huile qui sont dissous dans 150 ml d'acétone; on introduit ensuite dans cette solution 2,6 g d'acide sulfurique à 66° Bé; le précipité formé est isolé et séché pour donner 8 g d'hémisulfate du produit final qui fond vers 190°C. $[\alpha]_D^{20}$ = + 53,3° (C = 1,$CH_3OH$).

La réaction peut être effectuée à température ambiante; le rendement est identique lorsqu'elle est prolongée 4 jours.

EXEMPLE 6

Alpha-(tétrahydro-4,5,6,7thiéno[3,2-C]pyridyl-5)(chloro-2phényl)acétate de méthyle dextrogyre :

a) 20,5 g de (+)alpha-(thiényl-2éthylamino)-(chloro-2phényl)acétate de méthyle en solution dans 200 ml de chlorure de méthylène sont introduits sous agitation, en 25 minutes dans 40 ml d'une solution aqueuse d'aldéhyde formique à 30% (p/p). Après 3 heures d'agitation, la phase organique est décantée, lavée à l'eau, séchée et le solvant est évaporé. Le résidu est dissous dans 50 ml de chlorure de méthylène et la solution est introduite, à 60°C, dans 100ml de diméthylformamide anhydre contenant de l'acide chlorhydrique à concentration 6N. Après 1 h 30 à cette température les solvants sont éliminés par distillation sous pression réduite et le résidu est dissous dans 200 ml de chlorure de méthylène et 100 ml d'eau; on ajoute du bicarbonate de sodium pour libérer la base de son chlorhydrate, décante la phase organique, la sèche et concentre sous pression réduite. L'hémisulfate du produit cherché est préparé dans 150 ml d'acétone par action de 4,9 g d'acide sulfurique concentré (96%). On obtient ainsi 17 g de précipité. $[\alpha]_D^{20}$ = + 53° (C = 1,$CH_3OH$).

**Revendications**

**1.** Procédé de préparation de l'isomère dextrogyre du composé de formule

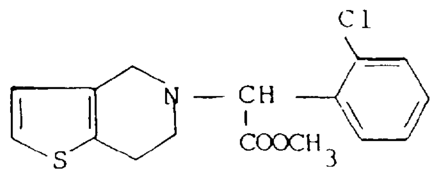

I

caractérisé en ce que l'on fait réagir un agent de formylation choisi parmi l'aldéhyde formique, son hydrate ou ses dérivés de polymérisation, les composés de formule $X\text{-}CH_2\text{-}Y$ dans laquelle X est un atome d'halogène, un groupe alkoxy en $C_1$-$C_4$, un groupe alkylthio en $C_1$-$C_4$ ou un groupe amino, et Y est un groupe alcoxy en $C_1$-$C_4$, un groupe alkylthio en $C_1$-$C_4$, un groupe amino, ou un groupe alcoxycarbonyle en $C_2$-$C_5$ ou phénoxycarbonyle ou les composés hétérocycliques de formule

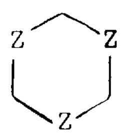

dans laquelle Z est O, NH ou S, sur l'isomère dextrogyre du composé de formule

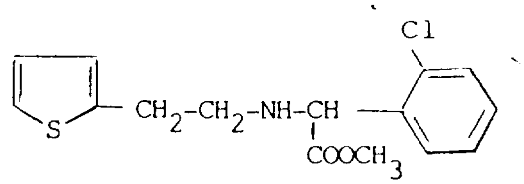

II

et, que l'on cyclise les composés formés en présence d'un acide.

2. Procédé selon la revendication 1, caractérisé en ce que l'agent de formylation est l'aldéhyde formique, son hydrate ou ses dérivés de polymérisation.

3. Procédé selon l'une des revendications 1 à 2, caractérisé en ce que l'acide est un acide minéral et qu'il est ajouté après la formylation.

4. Procédé selon la revendication 3, caractérisé en ce que la cyclisation est effectuée en présence d'un acide minéral fort et de diméthylformamide.

5. Procédé selon l'une des revendications 1 à 2, caractérisé en ce que l'acide est un acide organique.

6. Procédé selon la revendication 5, caractérisé en ce que l'acide est l'acide formique et qu'il est aussi utilisé comme solvant des réactions.

7. Procédé selon l'une des revendications 1 à 2, caractérisé en ce que l'acide est un acide fort, introduit sous forme de son sel avec le composé II.

8. Alpha-(thiényl-2 éthylamino)(chloro-2-phényl)acétate de méthyle dextrogyre et ses sels.

**Patentansprüche**

1. Verfahren zur Herstellung des rechtsdrehenden Isomeren der Verbindung der Formel

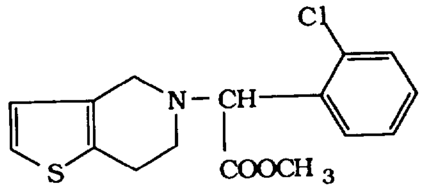

I

**dadurch gekennzeichnet**, daß man ein Formylierungsagens ausgewählt aus Formaldehyd, seinem Hydrat oder seinen Polymerisationsderivaten, Verbindungen der Formel X-$CH_2$-Y, worin X ein Halogenatom, eine $C_1$-$C_4$-Alkoxygruppe, eine $C_1$-$C_4$-Alkylthiogruppe oder eine Aminogruppe und Y eine $C_1$-$C_4$-Alkoxy-gruppe, eine $C_1$-$C_4$-Alkylthiogruppe, eine Aminogruppe oder eine $C_2$-$C_5$-Alkoxy-carbonylgruppe oder eine Phenoxycarbonylgruppe bedeuten, oder heterocyclischen Verbindungen der Formel

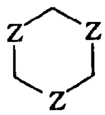

in der Z O, NH oder S bedeutet, mit dem rechtsdrehenden Isomeren der Verbindung der Formel

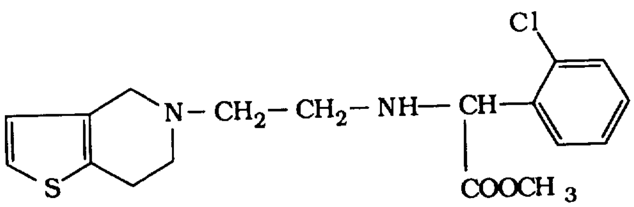

II

umsetzt und die gebildeten Verbindungen in Gegenwart einer Säure cyclisiert.

2. Verfahren nach Anspruch 1, **dadurch gekennzeichnet**, daß das Formylierungsagens Formaldehyd, sein Hydrat oder sein Polymerisationsderivat ist.

3. Verfahren nach einem der Ansprüche 1 bis 2, **dadurch gekennzeichnet**, daß die Säure eine Mineralsäure ist, die nach der Formylierung zugegeben wird.

4. Verfahren nach Anspruch 3, **dadurch gekennzeichnet**, daß die Cyclisierung in Gegenwart einer starken Mineralsäure und von Dimethylformamid durchgeführt wird.

5. Verfahren nach einem der Ansprüche 1 bis 2, **dadurch gekennzeichnet**, daß die Säure eine organische Säure ist.

6. Verfahren nach Anspruch 5, **dadurch gekennzeichnet**, daß die Säure Ameisensäure ist, die auch als Reaktionslösungsmittel verwendet wird.

7. Verfahren nach einem der Ansprüche 1 bis 2, **dadurch gekennzeichnet**, daß die Säure eine starke Säure ist, die in Form ihres Salzes mit der Verbindung II zugesetzt wird.

8. Rechtsdrehender α-(2-Thienyl-ethylamino)-(2-chlor-phenyl)-essigsäure-methylester und dessen Salze.

## Claims

1. Process for preparing the dextrorotatory isomer of the compound of formula

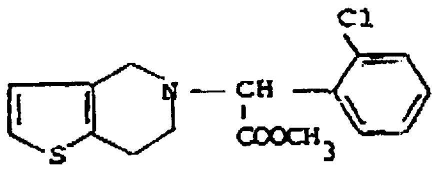

I

characterised in that a formylation agent selected from formic acid, the hydrate or the polymerisation derivatives thereof, the compounds of formula $X-CH_2-Y$ wherein X is a halogen atom, a $C_{1_4}$-alkoxy group, a $C_{1-4}$-alkylthio group or an amino group, and Y is a $c_{1-4}$-alkoxy group, a $C_{1-4}$-alkylthio group, an amino group or a $C_{2-5}$-alkoxycarbonyl or a phenoxycarbonyl group or the heterocyclic compounds of formula

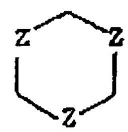

wherein Z is O, NH or S, is reacted with the dextrorotatory isomer of the compound of formula

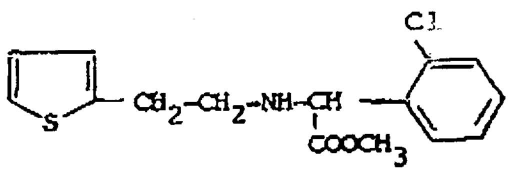

II

and the compounds formed are cyclised in the presence of an acid.

2. Process according to claim 1, characterised in that the formylation agent is formic aldehyde, the hydrate or the polymerisation derivatives thereof.

3. Process according to one of claims 1 and 2, characterised in that the acid is an inorganic acid and it is added after the formylation.

4. Process according to claim 3, characterised in that the cyclisation is carried out in the presence of a strong inorganic acid and dimethylformamide.

5. Process according to one of claims 1 and 2, characterised in that the acid is an organic acid.

6. Process according to claim 5, characterised in that the acid is formic acid and it is also used as solvent in the reactions.

7. Process according to one of claims 1 and 2, characterised in that the acid is a strong acid, used in the form of its salt with the compound II.

8. Dextrorotatory methyl-alpha-(2-thienyl-ethylamino)-(2-chlorophenyl)-acetate and the salts thereof.